Europäisches Patentamt

European Patent Office

Office européen des brevets

(19)

(11) Publication number: **0 001 259**
**B1**

(12) **EUROPEAN PATENT SPECIFICATION**

(45) Date of publication of patent specification: **13.10.82**

(21) Application number: **78100885.9**

(22) Date of filing: **14.09.78**

(51) Int. Cl.³: **C 07 D 301/03,**
**C 07 D 301/12,**
**C 07 D 303/04,**
**C 07 D 303/14**

(54) Oxidation of olefins to oxirane compounds with periodate compounds.

(30) Priority: **19.09.77 US 834610**
**19.09.77 US 834609**

(43) Date of publication of application:
**04.04.79 Bulletin 79/7**

(45) Publication of the grant of the patent:
**13.10.82 Bulletin 82/41**

(84) Designated Contracting States:
**DE FR GB NL**

(56) References cited:
**DE - A - 1 568 801**
**DE - A - 1 593 306**
**DE - A - 2 221 650**
**FR - A - 770 420**
**US - A - 3 436 409**
**US - A - 3 641 067**

**REAGENTS FOR ORGANIC SYNTHESIS vol. 4,**
**FIESER and FIESER; J. WILEY & Sons New York**
**SYNTHESIS (1974) pages 229—272 A. J.**
**FATIADI "New Applications of Periodic Acid and**

(73) Proprietor: **THE DOW CHEMICAL COMPANY**
**Dow Center 2030 Abbott Road Post Office Box**
**1967**
**Midland Michigan 48640 (US)**

(72) Inventor: **Hillis, James Edward**
**152 Dallas Drive**
**Angleton Brazoria Texas (US)**
Inventor: **Coker, William Perkins**
**103 Begonia**
**Lake Jackson Brazoria Texas (US)**
Inventor: **Lane, Robert Earl, Jr.**
**836 Sycamore**
**Lake Jackson Brazoria Texas (US)**

(74) Representative: **Casalonga, Alain et al,**
**BUREAU D.A. CASALONGA OFFICE JOSSE &**
**PETIT Baaderstrasse 12-14**
**D-8000 München 5 (DE)**

(56) References cited:
**Periodates in Organic and Bio-organic**
**Chemistry"**
**ULLMANNS ENCYKLOPAEDIE DER**
**TECHNISCHEN CHEMIE. 3rd edition, part 9,**
**1957, Urban & Schwarzenberg, München-Berlin**

## 0 001 259

Oxidation of olefins to oxirane compounds with periodate compounds

The present invention pertains to a new process for oxidizing olefins with periodate compounds to produce oxiranes, and to a process for preparing monobasic and dibasic alkali metal periodates from tribasic, tetrabasic or pentabasic alkali metal periodates.

Preparation of oxiranes from olefins

Olefins have been oxidized to form oxirane compounds by various methods such as by the dehydrochlorination of chlorohydrins, (P. P. McClellan, *Ind. Eng. Chem.*, Vol. 42, 12 (1950), pp. 2402—7), the reaction of hydroperoxides with olefins in the presence of catalysts (U.S. Patent 3,351,635 and R. Landau, et al., *Seventh World Petroleum Conference Proceedings*, Vol. 5, (1967), pp. 67—72). It is well known that ethylene can be converted to ethylene oxide by the direct vapor phase oxidation of ethylene with air or oxygen in the presence of a silver catalyst. Olefins have also been oxidized to form oxirane compounds by treatment with organic peracids such as peracetic acid, monoperphthalic acid and perbenzoic acid (U.S. Patent 3,341,556 and *Chem. Revs.* Vol. 45, (1949) pp. 16—25). Other olefin oxidation processes use derivatives of metals of the groups Ti, Zr, Hf, V, Nb, Ta, Mo, W, U with one or several oxygen atoms as described in German patent application DOS 2.221.650, peroxy boran as mentioned in German patent applications DOS 1.568.801 and DOS 1.593.306 metallic oxides as mentioned in French patent 770.420, thallic carboxylates (as described in USP 3.641.067, thallic oxide as disclosed in USP 3.436.409.

The present invention therefor provides an alternative method for the preparation of oxirane compounds from compounds containing olefinic unsaturation, said method having the following characteristics and/or advantages compared with known processes for preparing oxirane compounds:

1. A relatively high selectivity to the desired oxirane product;
2. A minimum of non-useful by-products;
3. No chlorine-containing co-product is produced;
4. Numerous olefins can be epoxidized;
5. The process can be conducted whether the periodate is in the liquid or solid state; and
6. The separation of the oxirane compounds from the periodate is relatively easy, especially when the periodate is a solid.

The present invention resides in a process for forming an oxirane compound, by oxidation of an olefin characterized by contacting a compound containing olefinic unsaturation or a mixture of such compounds with a periodate represented by the formulas $MIO_4$, $MH_4IO_6$, and $M_2H_3IO_6$, or their hydrates, wherein M is a monovalent metal or an ammonium group represented by the formula $R_4N^+$, wherein each R is independently hydrogen or a hydrocarbon group having from 1 to 8 carbon atoms, at a temperature, pressure and for a time sufficient to form said oxirane compound and thereafter recovering said oxirane compound by conventional means.

Suitable periodate compounds which can be employed herein as represented by the formulas above include, for example those compounds wherein M is lithium, sodium, potassium, rubidium, cesium, silver and an ammonium radical represented by the formula $R_4N^+$ wherein each $R_4$ is independently hydrogen or a hydrocarbon group having from 1 to 8 carbon atoms such as, for example, methyl, ethyl, propyl, butyl, octyl and phenyl.

Olefinically unsaturated materials which are epoxidized in accordance with the present invention include substituted and unsubstituted aliphatic and alicyclic olefins which may be hydrocarbons, esters, alcohols, ketones or ethers. Preferred compounds are those having from 2 to 30 carbon atoms, and preferably at least 3 carbon atoms. Illustrative olefins are ethylene, propylene, normal butylene, isobutylene, the pentenes, the methyl pentenes, the normal hexenes, the octenes, the dodecenes, cyclohexene, methyl cyclohexene, butadiene, styrene, methyl styrene, vinyl toluene, vinylcyclohexene and the phenyl cyclohexenes. Olefins having halogen, oxygen, sulfur, phosphorus and nitrogen substituents can be used. Such substituted olefins are illustrated by allyl alcohol, methallyl alcohol, cyclohexenol, diallyl ether, methyl methacrylate, methyl oleate, methyl vinyl ketone, allyl chloride, triallylphosphite, triallylphosphate and triallylamine. In general, all olefinic materials epoxidized by methods previously employed can be epoxidized in accordance with this process including olefinically unsaturated polymers having up to several thousand carbon atoms. Illustrative olefins are linseed oil, olive oil, soybean oil, cottonseed oil, tall oil glycerides, castor oil, corn oil, butyl-polyglycol esters of unsaturated fatty acids, liquid or solid polybutadiene, polyisoprene, and unsaturated copolymers of ethylene and propylene, including terpolymers thereof with cyclopentadiene.

The ratio of periodate compound to olefin is from about 1:1 to about 1:100, preferably from about 1:5 to about 1:20 moles of periodate:mole of unsaturation.

The temperature employed in the present process can vary from 100°C up to 400°C. The particular temperature which may advantageously be employed depends upon the particular periodate used. A suitable temperature can be easily determined in the laboratory by gradually raising the temperature of a mixture of the particular periodate and olefin until the corresponding oxirane compound is detected by the appropriate analytical method such as, for example, gas chromatography. In general, increasing the temperature beyond this point will increase the rate of formation of the oxirane compound.

Another method is to heat the particular periodate compound and observe that temperature at which significant quantities of oxygen are released.

Suitable temperatures for some of the periodate compounds employed in oxidizing propylene are as follows:

| Periodate compound | Temperature, °C |
|---|---|
| $LiIO_4$ | 119—175 |
| $Li_2H_3IO_6$ | 129—135 |
| $NaIO_4$ | 190—350 |
| $NaIO_4$* | 170—240 |
| $Na_2H_3IO_6$ | 180—214 |
| $KIO_4$ | 260—300 |
| $KIO_4$* | 221—285 |
| $RbIO_4$ | 271—280 |
| $CsIO_4$ | 158—300 |
| $AgIO_4$ | 165—350 |
| $Ag_2H_3IO_6$ | 109—149 |
| $Bu_4NIO_4$ | 123—140 |

*Supported on $Co_3O_4$.

The time of reaction is dependent upon the temperature and residence time of the olefin, usually from about 1 second to about 2 hours; but any length of time sufficient to provide a suitable conversion at the desired temperature can be employed.

Pressures which can be employed range from subatmospheric pressure to superatmospheric pressure, but generally pressures from 0,07 to 14,1 kg/cm² (1 psi absolute to 200 psig) are satisfactory.

The process is easily conducted by contacting the olefin with the periodate compound, either in the solid state or in solution. When the periodate compound is in a solution, the olefin can be bubbled through the solution of the periodate compound. When the periodate compound is in the solid state, the olefin can be passed through a bed of the solid material.

The resultant oxidized products can be separated from the periodate compounds by any suitable means such as filtration, distillation or combinations thereof.

The periodates can be employed in their natural form, e.g. crystalline solid or powder, and can be dissolved in suitable inert solvents or supported on suitable supports.

Suitable solvents are those which dissolve the periodate, but which do not react with the periodate at an appreciable rate under the reaction conditions employed. These may include such polar and non-polar solvents as water and aromatic and substituted amide types such as, for example, benzene, biphenyl, dimethylformamide, dimethylacetamide and mixtures thereof.

Suitable supports include inert inorganic solids such as, for example, alumina, silica, silicon carbide, molecular sieves, zirconia, cobaltous and cobaltic oxides (including cobalto-cobaltic oxide, $Co_3O_4$), ceric oxide ($CeO_2$) and Fuller's earth.

If desired, the olefins may be diluted with an inert material before coming into contact with the periodate. Suitable such inert materials include, for example, the well known inert gases such as nitrogen, helium and argon.

The periodate compounds if not commercially available can be prepared by the addition of a suitable metal hydroxide to periodic acid ($H_5IO_6$) in mole ratio of acid to hydroxide of from about 1:1 to about 2:1.

The periodate compounds can be prepared by the methods disclosed in "Analytical Applications of Periodic Acid and Iodic Acid and Their Salts" by G. Frederick Smith, 5th Ed. published by The G. Frederick Smith Chemical Co., Columbus Ohio, 1950, Chapter II.

Other ways of preparing the alkali metal periodate include the reaction of the metal iodide or iodate with $Cl_2$ or $Br_2$ in the presence of MOH. The periodate can be prepared by the oxidation of the iodate to a periodate with $K_2S_2O_8$, in the presence of MOH.

In the process for making epoxides from olefins and periodates, the periodates are converted to iodates. These iodates can then be converted to periodates by contacting the iodates with oxygen or an oxygen source such as air at temperatures of from about 150°C to about 800°C until the desired periodate is formed.

The desired oxirane compound can be separated from the other reaction products by any convenient means such as distillation, extraction, or combinations thereof.

3

Preparing alkali metal mono- and dibasic alkali metal periodates from tribasic, tetrabasic and pentabasic alkali metal periodates

Monobasic alkali metal periodates have been prepared by reacting tribasic alkali metal periodates with a strong mineral acid such as sulfuric acid or nitric acid. Such strong acids result in neutral by-products such as alkali metal nitrates or alkali metal sulfates. The process of preparing such periodates used in the present invention results in by-products such as alkali metal carbonates, bicarbonates or dilute caustic solutions which can be recovered and employed, if desired, in other processes such as, for example, precipitating magnesium hydroxide from seawater and neutralizing acidic process streams.

In the present invention there may be used a process for preparing an alkali metal monobasic, dibasic periodate or mixtures thereof, characterized by contacting an alkali metal tribasic, tetrabasic, pentabasic periodate or mixture thereof with a substance having a relative acidic value, pKa, of from 3 to 16 at a temperature of from 0°C to about 350°C and a pressure of from 1 atmosphere to 100 atmospheres for a time sufficient to form said alkali metal monobasic, dibasic periodate or mixtures thereof and wherein the molar ratio of periodate:said substance is from 1:1 to 1:1000.

Suitable tribasic alkali metal periodates which can be employed in the process of the present invention include, for example, $K_3H_2IO_6$, $Na_3H_2IO_6$, $Li_3H_2IO_6$, $Cs_3H_2IO_6$, $Rb_3H_2I_2O_{10}$, $Na_3IO_5$, $Cs_3IO_5$ and mixtures thereof.

Suitable pentabasic alkali metal periodates which can be employed in the process of the present invention include, for example, $Na_5IO_6$, $K_5IO_6$, $Li_5IO_6$, $Rb_5IO_6$, $Cs_5IO_6$ and mixtures thereof.

Suitable weakly acidic substances having relative acidic values, pKa of 3 to 16 which can be employed in the process of the present invention include, for example, $H_2O+CO_2$, $H_2CO_3$, $Na_2Cr_2O_7$, $K_2Cr_2O_7$, $WO_3$, $NaHCO_3$, $B_2O_3$, $H_3BO_4$ and mixtures thereof.

The following examples illustrate the present invention.

Example 1

A feed gas containing propylene with or without nitrogen as an inert diluent was passed through a glass tube in which five grams of solid potassium metaperiodate, $KIO_4$, was suspended on glass wool. The tube was heated and the effluent gas stream was analyzed by gas chromatography for the oxidation products. The products of the reaction were later verified by a combination of mass spectroscopy and gas chromatography. Periodic tests were made for carbon dioxide formation by bubbling the effluent through a saturated aqueous solution of calcium oxide. The results are given in the following Table I.

TABLE I

| Run No. | Feed (cc/min.) | | Temp. (°C) | $C_3H_6$ converted (%) | Selectivity (%) | | | | |
|---|---|---|---|---|---|---|---|---|---|
| | $C_3H_6$ | $N_2$ | | | Acet-aldehyde | Propylene Oxide | Propion-aldehyde | Acrolein | Acetone |
| 1 | 95 | — | 275 | — | 19 | 73 | 0.3 | 8 | 0.3 |
| 2 | 95 | — | 280 | — | 16 | 78 | 0.3 | 5 | 1 |
| 3 | 95 | — | 285 | — | 23 | 71 | — | 6 | — |
| 4 | 1 | 4 | 295 | 56 | 16 | 54 | 4 | 19 | 7 |
| 5 | 1 | 4 | 300 | 60 | 14 | 52 | 3 | 16 | 14 |
| 6 | 1 | 4 | 290 | 38 | 19 | 48 | 3 | 21 | 8 |

Comparative A

A stream of propylene and oxygen was passed over solid $AgIO_4$ and the effluent analyzed by gas chromatography for the formation of oxidation products as the temperature was increased. A 0.3% yield of propylene oxide was observed at 165°C and was found to have increased to 5% at 350°C. This example illustrates that direct oxidation in the presence of a periodate compound results in negligible formation of the desired oxirane.

Example 2

3 Grams of solid $KIO_4$ were finely ground and sprinkled onto a glass wool pad which was then rolled up and placed in a glass reaction tube in a tube furnace. The effluent was connected to a gas chromatograph through a gas sample valve. The reactor was purged with ethylene, the flow rate was established at 10 cc/min. and the temperature slowly raised. Periodic samples of the effluent were taken to identify the oxidation products which were formed. At 280°C, 60% of the oxidation product was found to be ethylene oxide with the remaining 40% being acetaldehyde. A similar experiment in which $NaIO_4$ was used also produced ethylene oxide as the major oxidation product.

Example 3

5 Grams of finely powdered $NaIO_4$ were placed in the reaction tube described in the previous example. A stream of nitrogen was bubbled through a container of allyl alcohol immediately before the vapors entered the top of the reaction tube. The effluent from the lower end of the reactor was passed through a carbon tetrachloride scrubber at 0°C to remove the oxidation products. The reactor was heated from room temperature to 240°C. Analysis of the trap at this time showed that glycidol had been produced from the reaction of allyl alcohol and sodium metaperiodate.

Example 4

The reaction between $NaIO_4$ and propylene under pressure was studied by placing approximately 1 g. of $NaIO_4$ in a 30.5 cm×0.95 cm (12″×3/8″) stainless steel tube. The pressures and flow rates were selected by adjustment of a regulator in the propylene line and a needle valve attached to the outlet of the reactor. Temperature of the reactor was controlled by placing it in a molten nitrate salt bath at 225°C. Analysis of the effluent was made by gas chromatography. Propylene at 15.0 psig (1.06 $kg/cm^2$) and an exit rate of 50 cc/min. gave a selectivity of propylene oxide of greater than 90% based on the resulting organic oxidation products. When the pressure was increased to 2.08 $kg/cm^2$ (29.5 psig) and the flow rate reduced to 20 cc/min., the selectivity to propylene oxide was found to also be in excess of 90%.

Example 5

2 Grams of the periodate being evaluated were placed in a glass tube reactor between glass wool plugs. The propylene flow was adjusted to either 15 cc/min. or 5.4 cc/min. The reactor was placed in a molten salt bath at 200°C and the temperature slowly increased until gas chromatographic analysis of the effluent showed that oxidation of the propylene was taking place. The results are given in the following Table II.

<div align="center">TABLE II</div>

| Run No. | Periodate | Temp. (°C) | Residence time (sec.) | $C_3H_6$ converted (%) | Selectivity (%) | | | |
|---|---|---|---|---|---|---|---|---|
| | | | | | Acet-aldehyde | Propylene Oxide | Propion-aldehyde+ Acrolein | Acetone |
| 1 | $NaIO_4$ | 238 | 12 | 19 | 2 | 98 | — | — |
| 2 | $NaIO_4$ | 238 | 12 | 11 | 3 | 97 | — | — |
| 3 | $NaIO_4$ | 238 | 12 | 15 | 5 | 95 | — | — |
| 4 | $KIO_4$ | 272 | 14 | 13 | 8 | 92 | — | — |
| 5 | $KIO_4$ | 280 | 14 | 16 | 8 | 92 | 0.5 | — |
| 6 | $KIO_4$ | 284 | 14 | 21 | 8 | 90 | 1 | 2 |
| 7 | $RbIO_4$ | 271 | 18 | 13 | 4 | 96 | — | — |
| 8 | $RbIO_4$ | 272 | 18 | 14 | 4 | 96 | — | 0.2 |
| 9 | $CsIO_4$ | 277 | 22 | 16 | 11 | 89 | 1 | — |
| 10 | $CsIO_4$ | 277 | 22 | 19 | 8 | 91 | 1 | — |
| 11 | $CsIO_4$ | 277 | 22 | 29 | 2 | 96 | 1 | 0.1 |

Example 6

A one gram sample of LiIO$_4$ was placed in a U-tube reactor between glass wool plugs. The flow of propylene was adjusted to 50 cc/min. and the reactor immersed in a molten salt bath at 150°C. The temperature was raised to 175°C, at which point gas chromatographic analysis of the effluent showed the organic oxidation products to be 92% propylene oxide and 8% acetone.

Example 7

Nitrogen was bubbled through cyclohexene and was then allowed to pass into a reactor containing Na$_2$H$_3$IO$_6$. As the temperature was increased, the effluent stream was analyzed by gas chromatography for the formation of oxidation products. At about 200°C, 18% of the cyclohexene in the feed gas was found to be converted to cyclohexene oxide.

Example 8

When propylene was bubbled through a solution of tetrabutylammonium periodate in biphenyl at atmospheric pressure, gas chromatographic analysis of the effluent showed the oxidation of propylene to propylene oxide would proceed with better than 95% selectivity at a reaction temperature between 120°C and 140°C.

Example 9

When LiIO$_4$·2.6H$_2$O (or LiH$_4$IO$_4$·0.6H$_2$O) was dissolved in either dimethylformamide or dimethylacetamide and propylene bubbled through the solutions, propylene oxide was found to be formed at reaction temperatures between 125°C and 140°C.

Example 10

A 10 gram sample of NaIO$_4$ was placed in a glass U-tube reactor. The nitrogen feed to the reactor was bubbled through a second U-tube, filled with styrene at 73°C, immediately before it entered the reactor. As the reactor was heated from 200°C to 240°C, the effluent was scrubbed through a toluene-filled trap at about 0°C. Analysis of this trap at the conclusion of the reaction showed styrene oxide to be the major reaction product.

Example 11

Trans-2-butene was passed through a glass U-tube containing 3.0 grams of NaIO$_4$ at a rate of approximately 15 cc/min. The effluent was scrubbed through a water filled trap which was cooled in an ice bath. Trans-2,3-epoxybutane was the major product observed in the water trap as the reactor was heated from 210°C to 245°C. In a similar experiment using cis-2-butene as the olefin, cis-2,3-epoxybutane was the major product observed in the water trap at a reaction temperature of 220°C.

Reference Example A

One gram of Li$_5$IO$_6$ was suspended in 50 cc of water and placed in an autoclave. After charging with 14.1 kg/cm$^2$ (200 psig), of CO$_2$ the autoclave and contents were heated to 130°C for 6 hours. When cool, a clear, colorless solution was removed and analyzed for periodate (86% of the I$^{7+}$ charge remained). The solution pH of 7.3 combined with the H$_5$IO$_6$-LiOH titration curve indicates the periodate exists as a mixture of the mono- and dibasic salts. In the lithium series of periodate salts, only the mono- and dibasic salts are water soluble. Thus, a clear solution alone indicates reduction to a mono- or dibasic salt.

Reference Example B

5.8 Grams of Na$_3$H$_2$IO$_6$ were placed in 200 ml of water and CO$_2$ was bubbled through the suspension. After 15 to 20 minutes, a clear solution was obtained at pH 11. As the CO$_2$ purge was continued (300 to 400 grams over 2 to 3 hours), a white precipitate formed. The final solution had a pH of 7.5 and yielded 2.5 grams of solids upon filtration. The infrared spectrum of this material indicated it was Na$_2$H$_3$IO$_6$.

Example 12

A sample of commercial Na$_3$H$_2$IO$_6$ which failed to produce 1,2-propylene oxide from propylene below 400°C was ground with one-half an equal molar amount of sodium dichromate in a mortar and pestle. 3 Grams of the dried-powdered mixture was then placed in a 2.54 cm (1 inch) i.d. glass reactor and heated under a propylene flow of 15 cc per minute. Propylene oxide was produced with good selectivity between 180°C and 245°C which indicated conversion of the tribasic periodate to mono- and/or dibasic periodate.

Example 13

Mixtures of commercial Na$_3$H$_2$IO$_6$ (5.9 grams, 0.02 mole) with an equimolar amount of WO$_3$, NaHCO$_3$, B$_2$O$_3$ or a two-fold molar amount of H$_3$BO$_3$ were separately slurried with water, dried in a vacuum oven and ground to a fine powder. Each of these mixtures was found to produce propylene

8

oxide when propylene was passed over them at 250°C and 20 cc per minute in a 2.54 cm (1 inch) i.d. glass reactor.

**Claim**

A process for forming an oxirane compound by oxidation of an olefin characterized by contacting a compound containing olefinic unsaturation or a mixture of such compounds with a periodate represented by one of the formulas $MIO_4$, $MH_4IO_6$, and $M_2H_3IO_6$, or their hydrates, wherein M is a monovalent metal or an ammonium group represented by the formula $R_4N^+$, wherein each R is independently hydrogen or a hydrocarbon group having from 1 to 8 carbon atoms, at a temperature, pressure and for a time sufficient to form said oxirane compound and thereafter recovering said oxirane compound by conventional means.

**Patentanspruch**

Verfahren zur Bildung einer Oxiran-Verbindung durch Oxidierung eines Olefin, dadurch gekennzeichnet, dass eine olefinisch ungesättigte Verbindung bzw. ein Gemisch solcher Verbindungen mit einem Perjodat gemäss einer der Formeln $MJO_4$, $MH_4JO_6$ und $M_2H_3JO_6$ bzw. ihrer Hydrate, worin M ein einwertiges Metall oder eine Ammoniumgruppe nach der Formel $R_4N^+$ ist, in der R unabhängig Wasserstoff oder eine Kohlenwasserstoffgruppe mit 1 bis 8 Kohlenstoffatomen ist, bei hinreichend hoher Temperatur, hinreichend hohem Druck und hinreichend lange Zeit in Kontakt gebracht wird, damit sich die genannte Oxiran-Verbindung bildet, und anschliessend diese Oxiran-Verbindung durch herkömmliche Mittel gewonnen wird.

**Revendication**

Procédé pour la formation d'un oxirane par oxydation d'une oléfine, caractérisé par la mise en contact d'un composé contenant l'insaturation oléfinique, ou un mélange de ces composés, avec un periodate représenté par une des formules $MIO_4$, $MH_4IO_6$ et $M_2H_3IO_6$, ou leurs hydrates, dans lesquelles M est un métal monovalent ou un groupe ammonium représenté par la formule $R_4N^+$, où chaque R est indépendamment de l'hydrogène ou un groupe hydrocarboné ayant de 1 à 8 atomes de carbone, à une température, sous une pression et pendant un temps, suffisants, pour former ledit oxirane, puis par la récuparation dudit oxirane par des moyens classiques.